Europäisches Patentamt

European Patent Office

Office européen des brevets

(11) Publication number: **0 331 934**
**A1**

## (12) EUROPEAN PATENT APPLICATION

(21) Application number: **89102569.4**

(22) Date of filing: **15.02.89**

(51) Int. Cl.⁴: **A61K 47/00 , A61K 37/24 , A61K 35/26**

(30) Priority: **11.03.88 IT 1973188**

(43) Date of publication of application:
**13.09.89 Bulletin 89/37**

(84) Designated Contracting States:
**AT BE CH DE ES FR GB GR IT LI LU NL SE**

(71) Applicant: **SCLAVO S.p.A.**
**Via Fiorentina 1**
**I-53100 Siena(IT)**

(72) Inventor: **Lattanzi, Filippo**
**Via Sansedoni, 9**
**Siena(IT)**

(74) Representative: **Cioni, Carlo et al**
**c/o ENIRICERCHE S.p.A. BRELID Via F.**
**Maritano 26**
**I-20097 San Donato Milanese(IT)**

(54) **New pharmaceutical compositions containing thymosin alpha 1.**

(57) A lyophilized pharmaceutical composition suitable for the preparation of parenteral dosage forms of thymosin $\alpha_1$ by reconstitution with an aqueous vehicle, characterized in that it comprises from 0.1 to 5 mg of human albumin per mg of thymosin $\alpha_1$.

EP 0 331 934 A1

## NEW PHARMACEUTICAL COMPOSITIONS CONTAINING THYMOSIN $\alpha_1$

The present invention relates to a new pharmaceutical composition comprising thymosin $\alpha_1$ as an active ingredient together with human albumin.

More particularly the present invention refers to a parenteral pharmaceutical composition, comprising thymosin $\alpha_1$ and human albumin.

Thymosin $\alpha_1$, is a polypeptide containing 28 amino acid residues, having the following sequence

$$Ac-Ser-Asp-Ala-Ala-Val-Asp-Thr-Ser-Ser-Glu-Ile-$$
$$-Val-Glu-Lys-Lys-Glu-Lys-Leu-Asp-Lys-Thr-Thr-$$
$$-Val-Glu-Glu-Ala-Glu-Asn-OH$$

Said octacosapeptide, which has been isolated, in 1976, from calf thymus gland (US-A-4,079,127), has potent immunostimulating activity (see for instance J. Biol. Chem. 1979, 254(3), pp.981-6, and Nachr. Chem. Tech. Lab., 1980, 28(4), pp.216-8). In particular, thymosin $\alpha_1$, when tested in vivo, afforded very interesting results in the protection from undesirable effects of the immunosuppressive agents employed in cancer therapy.

More particularly, thymosin $\alpha_1$, exerted a preventive activity against progression of leukemias in mice whose normal immune systems were severely damaged by treatment with cytostatic agents or X-irradiation, accelerating the replenishment and maturation of the hematopoietic cells (see for instance Cancer Immunol. Immunother. 1983, 15(2), pp. 78-83 and 108-13); furthermore, thymosin $\alpha_1$ also has a protective activity against opportunistic infections, being effective in protecting mice immunosuppressed by cytostatics from lethal infections with Candida albicans, Pseudomonas aeruginosa, and Serratia marcescens.

Presently, clinical trials which involve parenteral administration of this compound are in progress. For parenteral administration, strongly acidic peptides, like thymosin $\alpha_1$, are conventionally formulated as aqueous solutions containing a pharmaceutically acceptable buffer which is aimed at maintaining the pH of the injectable solution as close as possible to the physiological pH and stabilizing the active principle itself.

As far as thymosin $\alpha_1$ is concerned, said optimum pH value is between about 6 and about 8, and preferably from about 7 and about 7.5.

These conventional formulations however are not satisfactory when thymosin $\alpha_1$ is the active principle. In particular it has been noticed that by simply formulating thymosin $\alpha_1$ in aqueous solutions whose pH is in the range 7-7.5, the titer in active principle of the thus obtained dosage forms, steadily drops below the threshold of 90 %.

It has now been found and represents a first object of the present invention that it is possible to obtain a stable, highly acceptable formulation, which delivers the correct amount of drug in a reproducible, effective manner, by formulating thymosin $\alpha_1$ with a suitable amount of human albumin.

By the addition of human albumin, in fact, the titer in thymosin $\alpha_1$ of the new composition remains constant for a long period of time, thus ensuring correspondence of the administered dose to the prescribed one and repeatability of the selected dosages.

Furthermore, human albumin is compatible with the active principle as it does not alter thymosin $\alpha_1$ chemico-physical characteristics, absorption pattern or metabolic pathway.

More particularly, the amount of human albumin which affords the desired results ranges from about 0.1 to about 5 mg per mg of thymosin $\alpha_1$.

Higher amounts might also be used without providing additional advantages as a composition containing 5 mg of human albumin per mg of thymosin $\alpha_1$ is completely stable.

Human albumin employed in the preparation of the new composition is human albumin, purified for human use, meeting the standard requirements of the most recent Official Pharmacopeias.

In particular, either freeze-dried human serum albumin ready for use in humans, or the Albumin Injectable Solution, which consists of an aqueous solution containing from 5 to 26% (w/v) albumin, may be used.

The new pharmaceutical composition of the present invention, which is characterized in that it comprises thymosin $\alpha_1$ and human albumin, can be presented as a freeze-dried, soluble product, to be combined with an aqueous, pharmaceutically acceptable, solvent just prior to use or directly as an aqueous solution thereof ready for injection.

According to a preferred embodiment, the pharmaceutical composition of the present invention will be formulated as a freeze-dried product to be redissolved in sterile water for injection just before use, and according to a most preferred embodiment it will be presented as a single-dose administration unit.

To prepare a freeze-dried composition according to the present invention, thymosin $\alpha_1$ and albumin, in the suitably selected proportions, are dissolved in an aqueous sterile solution containing a pharmaceutically acceptable base and/or buffer which provides a pH within the range 6-8 and preferably within the range 7-7.5, and the obtained solution is cold-sterilized and freeze-dried under rigorously aseptical conditions. Bases which can suitably be employed to adjust the pH of the solution in the desired range, are for instance mineral bases such as sodium hydroxide or potassium hydroxide, while suitable buffers are, for instance, citrate buffer, monobasic potassium phosphate/dibasic potassium phosphate, and 2-amino-2-(hydroxymethyl)-1,3-propanediol (Tham).

The obtained solution may also contain other additives, e.g. antimicrobial agents and/or local anesthetics, as known in the art.

For the preparation of a single-dose administration unit, as known in the art, once an aqueous solution is obtained containing thymosin $\alpha_1$, human albumin, and all the other excipients, if any, in the desired proportions, said solution is transferred into the previously sterilized single-dose containers, by forcing a measured volume of the liquid, which provides for the selected amount of active principle, into each container through the orifice of a delivery tube which is introduced therein.

For a suitable medical practice, the amount of thymosin $\alpha_1$ per dosage unit will range from 0.5 to 4 mg.

The filled up dosage containers are then loaded into the freeze-drying chamber and freeze-dried until the product is dry. When the freeze-drying stage is complete, the containers are sealed by closing the opening with a rubber closure, while still in the freeze-drying chamber, under rigorously aseptical conditions. Rubber closures are finally held in place by means of aluminum caps which cover the closures and are crimped under the lid of the containers.

As for the concentration of thymosin $_1$ in the solution and the capacity of the dosage containers (interrelated parameters which can suitably be modified, depending on the desired amount of thymosin $\alpha_1$ in the finished single-dose container), these may vary within wide ranges, bearing in mind however that preferred single-dose administration units will contain an amount of thymosin $\alpha_1$ ranging from 0.5 to 4 mg, and that a suitable vial capacity, according to a conventional pharmaceutical practice, is generally from 1 to 5 ml and preferably from 1 to 2.5 ml.

For its use in therapy, the thus obtained freeze-dried formulation is redissolved in sterile water for injection and injected soon after its reconstitution.

A further object of the present invention is therefore the pharmaceutical single-dose administration unit which consists of a freeze-dried product comprising thymosin $\alpha_1$ as the active principle, and human albumin as the excipient or one of the excipients, and sterile water for injection for its reconstitution prior to use.

The following examples illustrate in further detail some representative formulations of the present invention and the process for their preparation.

It has to be understood that these examples are not to be interpreted as a limitation to the scopes of the invention because, on the basis of the information contained therein, any extrapolation to concentrations, excipients and preparation techniques falling within the above descriptive portion but different from those specifically exemplified, is possible for the average skilled technician.

Example 1

Thymosin $\alpha_1$ (66.0 g), human albumin (330.0 g) and citric acid monohydrate (1650 g) are charged in a glass vessel and dissolved, under stirring, in sterile water for injections (30 1). A 30 % NaOH solution is then added thereto to bring the pH to 7.4 and the volume is then adjusted to 33 1, by the addition of sterile water for injection.

The solution is then filtered through a sterilizing Millipore 0.22 $\mu$m filter, collecting the filtrate in a sterile glass flask kept in a sterile chamber.

With a suitable liquid filler, the solution is subdivided into previously sterilized vials (33000), which are then loaded into the freeze-drier and lyophilized.

When lyophilization is complete, the vials are sealed, while still in the freeze-drying chamber, by stoppering. Aluminum caps are then applied to the vials at the end of process line by means of a mechanical crimper.

Each vial contains a freeze-dried product comprising :

| thymosin $\alpha_1$ | 2.0 mg |
|---|---|
| human albumin | 10.0 mg |

Said lyophilizate is reconstituted with 1 or 2 ml of sterile Water for Injection USP just prior to use.

Example 2

An aqueous solution is prepared as described above but starting from 33.0 g of thymosin $\alpha_1$, 82.5 g of human albumin and 660.0 g citric acid monohydrate.

By following the procedure described in the preceding example, single-dose vials are obtained each containing 1.0 mg of thymosin $\alpha_1$ and 2.5 mg of human albumin.

Example 3

A formulation is prepared by following substantially the same procedure as in example 1 but using 1320 g of citric acid monohydrate and 3.3 g of 2-amino-2-(hydroxymethyl)-1,3-propanediol instead of 1650 g of citric acid monohydrate.

The composition, in each single vial, will contain :

| thymosin $\alpha_1$ | 2.0 mg |
|---|---|
| human albumin | 10.0 mg |
| citric acid monohydrate | 40.0 mg |
| 2-amino-2-(hydroxymethyl)-1,3-propanediol | 0.1 mg |
| sodium hydroxide up to pH 7.4 | |

Example 4

Comparative preparation

The same procedure as in example 1 has been followed with the only difference that no human albumin has been added.

The stability of the lyophilized products obtained in the foregoing examples 1 to 4 has been evaluated by determining the HPLC titer of the solutions obtained therefrom by reconstitution with sterile water for injection.

The results, as an average value of 5 determinations for each group, are reported in the following Table

| Lyophilizate of example no. | HPLC % titer |
|---|---|
| 1 | 98 |
| 2 | 97.5 |
| 3 | 98 |
| 4 | 88 |

4

**Claims**

1) A lyophilized pharmaceutical composition suitable for the preparation of parenteral dosage forms of thymosin $\alpha_1$ by reconstitution with an aqueous vehicle, characterized in that it comprises from 0.1 to 5 mg of human albumin per mg of thymosin $\alpha_1$.

2) The pharmaceutical composition of claim 1 which contains a pharmaceutically acceptable base and/or basic buffer, in such a concentration to bring the pH of the parenteral dosage form in the range 6-8.

3) The pharmaceutical composition of claim 2 wherein the pH is in the range 7-7.5

4) The pharmaceutical composition of claim 1 wherein the aqueous vehicle is water.

5) A parenteral dosage form of thymosin $\alpha_1$ comprising an aqueous solution of thymosin $\alpha_1$, a pharmaceutically acceptable base and/or basic buffer to bring the pH in the range 6-8 and from 0.1 to 5 mg of human albumin per mg of thymosin $\alpha_1$.

6) A single-dose administration unit which comprises a lyophilized pharmaceutical composition of claim 1 containing from 0.5 to 4 mg of thymosin $\alpha_1$.

7) A process for preparing a lyophilized pharmaceutical composition suitable for the preparation of parenteral dosage forms of thymosin $\alpha_1$ by reconstitution with an aqueous vehicle, which consists of preparing an aqueous solution comprising thymosin $\alpha_1$, from 0.1 to 5 mg of human albumin per mg of thymosin $\alpha_1$, and a pharmaceutically acceptable base and/or basic buffer to bring the pH in the range 6-8, and freeze-drying it.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 444 757 (H.R. STRAUSSER) * Column 10, lines 11-41 and in particular lines 24-26; column 4, lines 5-9; column 5, lines 34-38 * | 1-7 | A 61 K 47/00 A 61 K 37/24 A 61 K 35/26 |
| Y | EP-A-0 133 767 (GREEN CROSS) * Claims 1,6-10; page 3, lines 4-6,20-26; page 4, lines 5-12; page 6, example 10 * | 1-7 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 26-05-1989 | SCARPONI U. |